# EUROPEAN PATENT APPLICATION

(11) **EP 4 339 281 A1**
(43) Date of publication of application: **20.03.2024**
(21) Application number: 22820582.9
(22) Date of filing: 09.06.2022
(51) Int. Cl.: C12N 9/10, C12P 5/00, C12N 15/80

(54) **GERANYLGERANYL PYROPHOSPHATE SYNTHASE VARIANT AND TETRATERPENE USING SAME, PRECURSOR THEREOF, AND METHOD FOR PRODUCTION OF MATERIAL HAVING TETRATERPENE AS PRECURSOR**

(30) Priority: 09.06.2021 KR 20210074819
(71) Applicant: CJ Cheiljedang Corporation, Seoul 04560 (KR)
(72) Inventor: LEE, Peter, Seoul 04560 (KR); PARK, Hye Min, Seoul 04560 (KR); HA, Cheol Woong, Seoul 04560 (KR); LEE, Dong Pil, Seoul 04560 (KR)
(74) Representative: Weickmann & Weickmann PartmbB
(86) International application number: PCT/KR2022/008156
(87) International publication number: WO 2022/260455

(57) **Abstract**

The present disclosure relates to a geranylgeranyl pyrophosphate synthase variant; a polynucleotide encoding the variant; a vector including the polynucleotide; a microorganism including any one or more of the variant, the polynucleotide, and the vector; a method of producing tetraterpene, a precursor thereof, or a material having tetraterpene as a precursor using the same; a composition for producing tetraterpene, a precursor thereof, or a material having tetraterpene as a precursor; and use of the microorganism including any one or more of the variant, the polynucleotide, and the vector, for producing tetraterpene, a precursor thereof, and a material having tetraterpene as a precursor.

## Description

### [Technical Field]

The present disclosure relates to a geranylgeranyl pyrophosphate synthase variant; a polynucleotide encoding the variant; a vector including the polynucleotide; a microorganism including any one or more of the variant, the polynucleotide, and the vector; a method of producing tetraterpene, a precursor thereof, or a material having tetraterpene as a precursor using the same; a composition for producing tetraterpene, a precursor thereof, or a material having tetraterpene as a precursor; and use of the microorganism including any one or more of the variant, the polynucleotide, and the vector, for producing tetraterpene, a precursor thereof, and a material having tetraterpene as a precursor.

### [Background Art]

Terpenoids, which is a concept including carotenoids, exert various functions in plants and animals, and thus are used in a variety of industrial fields such as foods, feeds, *etc.* Terpenoids are known as representative substances showing plant medicinal properties, and like carotenoids, they are a type of terpenes. Among them, carotenoids such as beta-carotene are substances reported to have functions such as eliminating free radicals, acting as precursors for vitamin A in animals, enhancing the immune system of vertebrates, and reducing the risk of lung cancer.

However, despite these advantages, carotenoids, for example, beta-carotenes are not synthesized in the animal body or are synthesized in insufficient amounts. In addition, even though industrial production is attempted using mutated microorganisms (US Patent No. 7745170), it is still difficult to produce beta-carotene with high purity due to by-products.

With regard to by-products, geranylgeranyl pyrophosphate (GGPP, C20), which is a key precursor for carotenoid or terpenoid production, is produced by binding with farnesyl pyrophosphate (FPP, C15) and isopentenyl pyrophosphate (IPP, C5) by a geranylgeranyl pyrophosphate synthase enzyme in the isoprenoid biosynthetic pathway. However, squalene (C30) may be produced as a by-product from farnesyl pyrophosphate (FPP, C15) by squalene synthase (ERG9). Accordingly, there is a need to develop a method of increasing the production of GGPP, which is a key precursor for carotenoid or terpenoid biosynthesis, and a method of reducing the production of squalene, which is a competing pathway.

### [Disclosure]

### [Technical Problem]

The problem to be solved in the present disclosure is to provide a geranylgeranyl pyrophosphate synthase variant and a method of producing tetraterpene, a precursor thereof, and a material having tetraterpene as a precursor using the same.

### [Technical Solution]

An object of the present disclosure is to provide a geranylgeranyl pyrophosphate synthase variant, wherein any one or more of amino acids corresponding to positions 29, 43, 90, 103, 122, and 141 from the N-terminus of an amino acid sequence of SEQ ID NO: 1 are substituted with another amino acid.

Another object of the present disclosure is to provide a polynucleotide encoding the variant.

Still another object of the present disclosure is to provide a vector including the polynucleotide.

Still another object of the present disclosure is to provide a microorganism including any one or more of the variant, the polynucleotide, and the vector.

Still another object of the present disclosure is to provide a method of producing tetraterpene, a precursor thereof, and a material having tetraterpene as a precursor, the method including culturing the microorganism in a medium.

Still another object of the present disclosure is to provide a composition for producing tetraterpene, a precursor thereof, and a material having tetraterpene as a precursor, the composition including the variant, the vector, the microorganism, or the culture of the microorganism.

Still another object of the present disclosure is to provide use of any one or more of the variant, the polynucleotide, the vector, and the microorganism, for producing tetraterpene, a precursor thereof, and a material having tetraterpene as a precursor.

### [Advantageous Effects]

A microorganism expressing a geranylgeranyl pyrophosphate synthase variant of the present disclosure may effectively produce tetraterpene, a precursor of tetraterpene, or a material having tetraterpene as a precursor, as compared to a strain not expressing the same.

### [Detailed Description of Preferred Embodiments]

Hereinafter, the present disclosure will be described in detail as follows. Meanwhile, each description and embodiment disclosed in this disclosure may also be applied to other descriptions and embodiments. Further, all combinations of various elements disclosed in this disclosure fall within the scope of the present disclosure. Further, the scope of the present disclosure is not limited by the specific description described below.

An aspect of the present disclosure provides a geranylgeranyl pyrophosphate synthase variant, wherein any one or more of amino acids corresponding to positions 29, 43, 90, 103, 122, and 141 from the N-terminus of an amino acid sequence of SEQ ID NO: 1 are substituted with another amino acid.

The geranylgeranyl pyrophosphate synthase variant refers to a variant, wherein any one or more of amino acids corresponding to positions 29, 43, 90, 103, 122, and 141 from the N-terminus of SEQ ID NO: 1 are substituted with another amino acid in a polypeptide having geranylgeranyl pyrophosphate synthase activity or in a geranylgeranyl pyrophosphate synthase.

As used herein, the "geranylgeranyl pyrophosphate synthase (GGS1)" is an enzyme capable of catalyzing synthesis of geranylgeranyl pyrophosphate (GGPP) from farnesyl pyrophosphate (FPP).

The geranylgeranyl pyrophosphate synthase of the present disclosure may be a geranylgeranyl pyrophosphate synthase or a polypeptide having geranylgeranyl pyrophosphate synthase activity to which modification is applied in order to prepare the geranylgeranyl pyrophosphate synthase variant provided in the present disclosure. Specifically, the geranylgeranyl pyrophosphate synthase may be a naturally occurring polypeptide or a wild-type polypeptide, and may be a mature polypeptide thereof, and may include a variant or functional fragment thereof, but any one may be included without limitation as long as it may be a parent of the geranylgeranyl pyrophosphate synthase variant of the present disclosure.

In the present disclosure, the geranylgeranyl pyrophosphate synthase may be, but is not limited to, a polypeptide of SEQ ID NO: 1. Further, the geranylgeranyl pyrophosphate synthase may be a polypeptide having about 80%, 85%, 90%, 95%, 96%, 97%, 98%, or 99% or more sequence identity to the polypeptide of SEQ ID NO: 1, and any one may fall within in the scope of the geranylgeranyl pyrophosphate synthase without limitation, as long as it may have activity identical or corresponding to that of the polypeptide consisting of the amino acid sequence of SEQ ID NO: 1.

The sequence of the geranylgeranyl pyrophosphate synthase of the present disclosure may be available in the NCBI GenBank, *etc.,* which is a known database. Specifically, the geranylgeranyl pyrophosphate synthase may be a polypeptide encoded by *ggs1* gene, but is not limited thereto.

As used herein, the "variant" refers to a polypeptide which has an amino acid sequence different from that of the variant before modification by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased as compared to that of the polypeptide before being varied. Further, some variants may include variants in which one or more portions such as an N-terminal leader sequence or a transmembrane domain have been removed. Other variants may include variants in which a portion of the N-and/or C-terminus has been removed from the mature protein. The term "variant" may be used interchangeably with terms such as modification, modified polypeptide, modified protein, mutant, mutein, and divergent and is not limited thereto as long as it is a term used with the meaning of variation.

Further, the variant may include deletions or additions of amino acids that have minimal effect on the properties and secondary structure of the polypeptide. For example, a signal (or leader) sequence that is co-translationally or post-translationally involved in the protein translocation may be conjugated to the N-terminus of the variant. The variant may be conjugated with other sequences or linkers so as to be identified, purified, or synthesized.

The variant of the present disclosure may be a geranylgeranyl pyrophosphate synthase variant in which any one or more amino acids of amino acids corresponding to positions 29, 43, 90, 103, 122, and 141 from the N-terminus of the amino acid sequence of SEQ ID NO: 1 are substituted with another amino acid. Specifically, amino acids at one or more, two or more, three or more, four or more, five or more, or six or more positions of positions 29, 43, 90, 103, 122, and 141 may be substituted, but are not limited thereto.

In the polypeptide having geranylgeranyl pyrophosphate synthase activity or geranylgeranyl pyrophosphate synthase which is a parent of the variant, the amino acid corresponding to position 29 from the N-terminus of the amino acid sequence of SEQ ID NO: 1 may be asparagine; the amino acid corresponding to position 43 may be phenylalanine; the amino acid corresponding to position 90 may be leucine; the amino acid corresponding to position 103 may be methionine; the amino acid corresponding to position 122 may be isoleucine; and/or the amino acid corresponding to position 141 may be arginine, but is not limited thereto.

In one embodiment, the variant may include one or more substitutions of a substitution of the amino acid corresponding to position 29 from the N-terminus of the amino acid sequence of SEQ ID NO: 1 with an amino acid other than asparagine; a substitution of the amino acid corresponding to position 43 with an amino acid other than phenylalanine; a substitution of the amino acid corresponding to position 90 with an amino acid other than leucine; a substitution of the amino acid corresponding to position 103 with an amino acid other than methionine; a substitution of the amino acid corresponding to position 122 with an amino acid other than isoleucine; and a substitution of the amino acid corresponding to position 141 with an amino acid other than arginine, but is not limited thereto.

The "another amino acid" is not limited as long as it is an amino acid other than the amino acid before substitution. Meanwhile, in the present disclosure, when expressed as 'a specific amino acid is substituted', it is obvious that the amino acid is substituted with an amino acid that is different from the amino acid before substitution, unless otherwise described as being substituted with a different amino acid.

The substitution with another amino acid may be substitution with a nonpolar amino acid, a polar amino acid, or a positively charged (basic) amino acid. Specifically, the nonpolar amino acid may be selected from the group consisting of glycine, alanine, valine, leucine, isoleucine, methionine, and proline, but is not limited thereto. The polar amino acid may be used interchangeably with a hydrophilic amino acid, and may be selected from the group consisting of serine, threonine, cysteine, tyrosine, asparagine and glutamine, and the positively charged (basic) amino acid may be selected from the group consisting of arginine, lysine, and histidine, but is not limited thereto.

Specifically, the variant of the present disclosure may be a variant in which any one or more of the amino acids corresponding to positions 29, 43, 90, 103, 122, and 141 in the amino acid sequence of SEQ ID NO: 1 which is a reference protein may be substituted with an amino acid different from the amino acid before substitution, among nonpolar amino acids, polar amino acids, or positively charged (basic) amino acids, but is not limited thereto. The nonpolar amino acid may be isoleucine (Ile) or valine (Val), the polar amino acid may be threonine (Thr), and the positively charged amino acid may be arginine (Arg) or lysine (Lys), but is not limited thereto.

Further, another amino acid may be any one selected from the group consisting of threonine, isoleucine, arginine, valine, and lysine, but is not limited thereto.

In one embodiment, in the variant of the present disclosure, any one or more amino acids of amino acids corresponding to positions 29, 43, 90, 103, 122, and 141 of the amino acid sequence of SEQ ID NO: 1 may be substituted with any one selected from the group consisting of threonine, isoleucine, arginine, valine, and lysine, but is not limited thereto.

In one embodiment, the variant of the present disclosure may comprise a substitution selected from the group consisting of a substitution of the amino acid corresponding to position 29 of the amino acid sequence of SEQ ID NO: 1 with threonine; a substitution of the amino acid corresponding to position 43 with isoleucine; a substitution of the amino acid corresponding to position 90 with arginine; a substitution of the amino acid corresponding to position 103 with isoleucine; a substitution of the amino acid corresponding to position 122 with valine; and a substitution of the amino acid corresponding to position 141 with lysine, and combinations thereof, but is not limited thereto.

As used herein, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, the "corresponding region" generally refers to a similar or corresponding position in a related protein or a reference protein.

For example, an arbitrary amino acid sequence is aligned with SEQ ID NO: 1, and based on this, each amino acid residue of the amino acid sequence may be numbered with reference to the numerical position of the amino acid residue corresponding to the amino acid residue of SEQ ID NO: 1. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), *etc.* may be used, but are not limited thereto, and a sequence alignment program, a pairwise sequence comparison algorithm, *etc.,* which known in the art, may be appropriately used.

In one embodiment, the variant of the present disclosure may comprise substitution of any one or more amino acids of the amino acids corresponding to positions 29, 43, 90, 103, 122, and 141 of SEQ ID NO: 1 with another amino acid while having 80%, 85%, 90%, 95%, 96%, 97%, 98%, or 99% or more sequence identity to the polypeptide of SEQ ID NO: 1.

In one embodiment, the variant of the present disclosure may include an amino acid sequence having at least 80%, 85%, 90%, 95%, 96%, 97%, 98%, 99%, 99.5%, 99.7%, or 99.9% or more homology or identity to an amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, or SEQ ID NO: 17.

Specifically, the variant of the present disclosure may have, may comprise, or may consist of the amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, or SEQ ID NO: 17, or may essentially consist of the above amino acid sequence.

In one embodiment, the variant of the present disclosure may include an amino acid sequence comprising a substitution of any one or more amino acids of the amino acids corresponding to positions 29, 43, 90, 103, 122, and 141, based on the amino acid sequence of SEQ ID NO: 1, with another amino acid and having at least 80%, 85%, 90%, 95%, 96%, 97%, 98%, 99%, 99.5%, 99.7% or 99.9% or more homology or identity to the amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, or SEQ ID NO: 17.

In one embodiment, the variant of the present disclosure may be any one or more selected from a polypeptide including the amino acid sequence represented by SEQ ID NO: 3, in which methionine which is the amino acid corresponding to position 103 of the amino acid sequence of SEQ ID NO: 1 is substituted with isoleucine; a polypeptide including the amino acid sequence represented by SEQ ID NO: 5, in which phenylalanine which is the amino acid corresponding to position 43 of the amino acid sequence of SEQ ID NO: 1 is substituted with isoleucine; a polypeptide including the amino acid sequence represented by SEQ ID NO: 7, in which asparagine which is the amino acid corresponding to position 29 of the amino acid sequence of SEQ ID NO: 1 is substituted with threonine, and leucine which is the amino acid corresponding to position 90 is substituted with arginine; a polypeptide including the amino acid sequence represented by SEQ ID NO: 9, in which asparagine which is the amino acid corresponding to position 29 of the amino acid sequence of SEQ ID NO: 1 is substituted with threonine; a polypeptide including the amino acid sequence represented by SEQ ID NO: 11, in which leucine which is the amino acid corresponding to position 90 of the amino acid sequence of SEQ ID NO: 1 is substituted with arginine; a polypeptide including the amino acid sequence represented by SEQ ID NO: 13, in which isoleucine which is the amino acid corresponding to position 122 of the amino acid sequence of SEQ ID NO: 1 is substituted with valine, and arginine which is the amino acid corresponding to position 141 is substituted with lysine; a polypeptide including the amino acid sequence represented by SEQ ID NO: 15, in which isoleucine which is the amino acid corresponding to position 122 of the amino acid sequence of SEQ ID NO: 1 is substituted with valine; and a polypeptide including the amino acid sequence represented by SEQ ID NO: 17, in which arginine which is the amino acid corresponding to position 141 of the amino acid sequence of SEQ ID NO: 1 is substituted with lysine, but is not limited thereto.

It is also apparent that a variant having an amino acid sequence having deletion, modification, substitution, conservative substitution, or addition of some sequence also falls within the scope of the present disclosure as long as the amino acid sequence has such a homology or identity and exhibits efficacy corresponding to that of the variant of the present disclosure.

Examples thereof include those having sequence addition or deletion that does not alter the function of the variant of the present disclosure at the N-terminus, C-terminus of the amino acid sequence, and/or inside the amino acid sequence, naturally occurring mutation, silent mutation, or conservative substitution.

The "conservative substitution" means substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of proteins or polypeptides.

As used herein, the term 'homology' or 'identity' refers to the degree of similarity between two given amino acid sequences or nucleotide sequences and may be expressed as a percentage. The terms 'homology and identity' may be often used interchangeably.

The sequence homology or identity of conserved polynucleotides or polypeptides may be determined by a standard alignment algorithm, and default gap penalties established by a program to be used may be used together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, homology, similarity, or identity may be determined using BLAST or ClustalW of the National Center for Biotechnology Information.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may include: (1) a binary comparison matrix (containing a value 1 for identity and a value 0 for non-identity) and the weighted comparison matrix of Gribskov et a/(1986) Nucl. Acids Res. 14: 6745 as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

In one embodiment, the variant of the present disclosure may have the geranylgeranyl pyrophosphate synthase activity. In one embodiment, the variant of the present disclosure may have activity that increases ability to produce tetraterpene, a precursor of tetraterpene, or a material having tetraterpene as a precursor, as compared to a wild-type or an unmodified geranylgeranyl pyrophosphate synthase. In one embodiment, the variant of the present disclosure may have activity that decreases a production level of by-products in the production pathway of tetraterpene, a precursor of tetraterpene, or a material having tetraterpene as a precursor, as compared to a wild-type or unmodified geranylgeranyl pyrophosphate synthase. In one embodiment, the variant of the present disclosure may have activity that decreases a production level of squalene, as compared to a wild-type or unmodified geranylgeranyl pyrophosphate synthase. In one embodiment, the variant of the present disclosure may have enhanced activity, as compared to the wild-type or unmodified geranylgeranyl pyrophosphate synthase, but is not limited thereto.

Another aspect of the present disclosure provides a polynucleotide encoding the variant of the present disclosure.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant having the geranylgeranyl pyrophosphate activity.

The polynucleotide encoding the variant of the present disclosure may include a nucleotide sequence encoding the amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, or SEQ ID NO: 17. In one embodiment, the polynucleotide of the present disclosure may have or may include a sequence of SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18. Further, the polynucleotide of the present disclosure may consist of or may essentially consist of a sequence of SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure may have or may include a nucleotide sequence having 80% or more, 85% or more, 90% or more, 95% or more, 96% or more, 97% or more, 98% or more, and less than 100% homology or identity to the sequence of SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18, or may consist of or may essentially consist of a nucleotide sequence having 80% or more, 85% or more, 90% or more, 95% or more, 96% or more, 97% or more, 98% or more, and less than 100% homology or identity to the sequence of SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18, but is not limited thereto.

In this regard, in SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18 of the sequence having homology or identity, codons encoding amino acids corresponding to positions 29, 43, 90, 103, 122, and 141 of the present disclosure may be any one of codons encoding threonine, isoleucine, arginine, valine, or lysine.

Further, the polynucleotide of the present disclosure may include a probe that may be prepared from a known gene sequence, for example, may include a sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, *i.e.,* polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1X SSC, 0.1% SDS, specifically, 60°C, 0.1X SSC, 0.1% SDS, more specifically 68°C, 0.1X SSC, 0.1 % SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also include substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook *et al.,* supra).

Still another aspect of the present disclosure provides a vector including the polynucleotide of the present disclosure. The vector may be an expression vector for expressing the polynucleotide in a host cell, but is not limited thereto.

The vector of the present disclosure may include a DNA construct including a nucleotide sequence of a polynucleotide encoding an interest polypeptide which is operably linked to a suitable expression regulatory region (or expression control sequence) so that the interest polypeptide may be expressed in a suitable host. The expression regulatory region may include a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or a cosmid vector, and pDC system, pBR system, pUC system, pBluescript II system, pGEM system, pTZ system, pCL system, pET system, *etc.* may be used as a plasmid vector. Specifically, pDC, pDCM2 (Korean Patent Publication No. 10-2020-0136813), pACYC177, pACYC184, pCL, pECCG117, pUC19, pBR322, pMW118, pCC1BAC, pIMR53 vector, *etc.* may be used.

For example, a polynucleotide encoding an interest polypeptide may be inserted into a chromosome through a vector for intracellular chromosome insertion. Insertion of the polynucleotide into the chromosome may be performed by any method known in the art, for example, homologous recombination, but is not limited thereto. The vector may further include a selection marker for the confirmation of chromosome insertion. The selection marker is for selecting the cells transformed with vectors, *i.e.,* for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector including a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell and located inside or located outside the chromosome as long as it may be expressed in the host cell. Further, the polynucleotide includes DNA and/or RNA encoding an interest polypeptide. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the desired variant of the present disclosure.

Still another aspect of the present disclosure provides a microorganism including any one or more of the variant of the present disclosure; the polynucleotide encoding the variant; and the vector including the polynucleotide

As used herein, the term "microorganism" or "strain" includes all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism including genetic modification for production of a polypeptide, protein, or product of interest.

The microorganism of the present disclosure may be a microorganism including any one or more of the variant of the present disclosure, the polynucleotide of the present disclosure, and the vector including the polynucleotide of the present disclosure; a microorganism modified to express the variant of the present disclosure or the polynucleotide of the present disclosure; a microorganism expressing the variant of the present disclosure or the polynucleotide of the present disclosure; or a microorganism (e.g., a recombinant microorganism) having the activity of the variant of the present disclosure, but is not limited thereto. The microorganism may be a microorganism which is modified to further include polynucleotides encoding lycopene cyclase/phytoene synthase (crtYB) and phytoene desaturase (crtl) proteins, thereby exhibiting activities of the proteins, or a microorganism in which activities of the proteins are enhanced. The lycopene cyclase/phytoene synthase or phytoene desaturase may be a protein derived from *Xanthophyllomyces dendrorhous,* but is not limited thereto. In a specific embodiment, the polynucleotide encoding the lycopene cyclase/phytoene synthase or phytoene desaturase may have or may include a sequence of SEQ ID NO: 27 or SEQ ID NO: 28, respectively. In the polynucleotide, various modifications may be made in the coding region as long as the amino acid sequence is not changed in consideration of codon degeneracy or codons preferred in microorganisms that are intended to express the variant of the present disclosure. Specifically, the polynucleotide may have or may include a nucleotide sequence having 80% or more, 85% or more, 90% or more, 95% or more, 96% or more, 97% or more, 98% or more, and less than 100% homology or identity to the sequence of SEQ ID NO: 27 or SEQ ID NO: 28, or may consist of or may essentially consist of a nucleotide sequence having 80% or more, 85% or more, 90% or more, 95% or more, 96% or more, 97% or more, 98% or more, and less than 100% homology or identity to the sequence of SEQ ID NO: 27 or SEQ ID NO: 28, but is not limited thereto.

The microorganism of the present disclosure may produce tetraterpene, a precursor of tetraterpene, or a material having tetraterpene as a precursor, and may have a reduced production of by-products which are generated in the tetraterpene production pathway or may have a reduced production of squalene, but is not limited thereto.

As used herein, the term "tetraterpene" refers to a polymer (C40) in which eight isoprenes are polymerized, among terpenes, and may include tetraterpene-based substances. The terpene is a polymer of isoprene units, and a terpene prepared by polymerizing two isoprene (C5) units is a monoterpene (C10), and a terpene prepared by polymerizing three isoprene units is a sesquiterpene (C15), and such terpenes may be classified according to the number of polymerized isoprene units.

Specifically, the tetraterpene may be carotenoid, but is not limited thereto.

In the present disclosure, the precursor of tetraterpene may be geranylgeranyl pyrophosphate (GGPP), but is not limited thereto.

In the present disclosure, the material having tetraterpene as a precursor may be any one selected from the group consisting of retinal and retinol, but is not limited thereto.

As used herein, the "tetraterpenoid" is a modified tetraterpene. The tetraterpenoid may be a tetraterpene to which functional group(s) are bound.

As used herein, the "carotenoid" refers to tetraterpene or derivatives thereof that give colors such as yellow in fruits and vegetables, and may be used interchangeably with the above tetraterpene and tetraterpenoid.

Specifically, the carotenoid may be any one or more selected from the group consisting of xanthophyll, carotene, alpha-carotene, beta-carotene, gamma-carotene, lutein, lycopene, zeaxanthin, capsanthin, canthaxanthin, and astaxanthin, but is not limited thereto.

In the present disclosure, the precursor of carotenoid may be geranylgeranyl pyrophosphate (GGPP), but is not limited thereto.

In the present disclosure, the material having carotenoid as a precursor may be any one or more selected from the group consisting of retinal and retinol, but is not limited thereto.

As used herein, the "beta-carotene" is one of carotenoid-based substances, and has beta-rings at both ends of the molecule, among the carotenes.

In the present disclosure, the precursor of beta-carotene may be any one or more selected from the group consisting of phytoene, phytofluene, lycopene, and gamma-carotene, but is not limited thereto.

In the present disclosure, the material having beta-carotene as a precursor may be any one or more selected from the group consisting of retinal, retinol, and vitamin A, but is not limited thereto.

The tetraterpene, the precursor of tetraterpene, or the material having tetraterpene as a precursor may be carotenoid, a precursor of carotenoid, or a material having carotenoid as a precursor, or may be beta-carotene, a precursor of beta-carotene, or a material having beta-carotene as a precursor, but is not limited thereto.

In the present disclosure, any one or more of the variant, the polynucleotide, the vector, and the microorganism may be for producing tetraterpene, and the microorganism may produce tetraterpene, and may produce the precursor of tetraterpene or the material having tetraterpene as a precursor.

In the present disclosure, any one or more of the variant, the polynucleotide, the vector, and the microorganism may be for producing carotenoid, and the microorganism may produce carotenoid, and may produce the precursor of carotenoid or the material having carotenoid as a precursor.

In the present disclosure, any one or more of the variant, the polynucleotide, the vector, and the microorganism may be for producing beta-carotene, and the microorganism may produce beta-carotene, and may produce the precursor of beta-carotene or the material having beta-carotene as a precursor.

In one embodiment, geranylgeranyl pyrophosphate (GGPP) is produced by geranylgeranyl pyrophosphate synthase in the isoprenoid biosynthetic pathway, and therefore, the microorganism may also produce geranylgeranyl pyrophosphate.

In the present disclosure, by-products generated in the tetraterpene production pathway refer to materials other than tetraterpene, the precursor of tetraterpene, and the material having tetraterpene as a precursor, specifically, squalene, but is not limited thereto. The beta-carotene may be produced in the carotenoid or isoprenoid production pathway. In this process, two molecules of farnesyl pyrophosphate (FPP, C15) are consumed by squalene synthase (ERG9), and squalene (C30) may be generated as the by-product.

As used herein, the "squalene" is an unsaturated hydrocarbon (C₃₀H₅₀) and is a material which is also used in the biosynthesis of steroid hormones, vitamin D, *etc.* The microorganism of the present disclosure may have reduced by-products which are generated in the tetraterpene production pathway, and specifically, may have reduced squalene production, but is not limited thereto.

The microorganism or strain of the present disclosure may be a microorganism naturally having the geranylgeranyl pyrophosphate synthase or the tetraterpene-producing ability, or a microorganism obtained by introducing the variant of the present disclosure or the polynucleotide encoding the same (or the vector including the polynucleotide) into and/or by providing ability to produce the tetraterpene, the precursor of tetraterpene, or the material having tetraterpene as a precursor for a parent strain without the geranylgeranyl pyrophosphate synthase or tetraterpene-producing ability, but is not limited thereto.

For example, the microorganism or strain of the present disclosure may be a cell or microorganism which is transformed with the polynucleotide of the present disclosure or the vector including the polynucleotide encoding the variant of the present disclosure to express the variant of the present disclosure. With respect to the objects of the present disclosure, the microorganism or strain of the present disclosure may include any microorganism that includes the variant of the present disclosure to produce tetraterpene, the precursor tetraterpene, or the material having tetraterpene as a precursor. For example, the microorganism or strain of the present disclosure may be a recombinant strain in which the polynucleotide encoding the variant of the present disclosure is introduced into a natural wild-type microorganism or a tetraterpene-producing microorganism to express the geranylgeranyl pyrophosphate synthase variant and to have an increased ability to produce tetraterpene, the precursor of tetraterpene, or the material having tetraterpene as a precursor. The recombinant strain may be a microorganism having an increased ability to produce tetraterpene, the precursor of tetraterpene, or the material having tetraterpene as a precursor, as compared to a natural wild-type microorganism or a microorganism in which the geranylgeranyl pyrophosphate synthase is unmodified (i.e., a wild-type geranylgeranyl pyrophosphate synthase-expressing microorganism), but is not limited thereto.

For example, the recombinant strain may have about 1% or more, specifically, about 3%, about 5% or more increased ability, as compared to the ability to produce tetraterpene, the precursor of tetraterpene, or the material having tetraterpene as a precursor of the parent strain before modification or the unmodified microorganism. However, as long as the recombinant strain has an increased ability of + value, as compared to the production ability of the parent strain before modification or the unmodified microorganism, it is not limited thereto.

For another example, the recombinant strain may have production of by-products generated in the tetraterpene production pathway, which is reduced by about 85% or less, about 80% or less, about 75% or less, about 70% or less, about 65% or less, about 60% or less, about 55% or less, about 50% or less, about 30% or less, or about 10% or less, as compared to the parent strain before modification or the unmodified microorganism, or may not produce by-products, but is not limited thereto.

As used herein, the term "about" refers to a range which includes all of ±0.5, ±0.4, ±0.3, ±0.2, ±0.1, *etc.,* and includes all of the values that are equivalent or similar to those following the term "about", but the range is not limited thereto.

As used herein, the term "unmodified microorganism" does not exclude strains including mutation that may occur naturally in microorganisms, and may be a wild-type strain or a natural strain itself or may be a strain before the trait is changed by genetic variation due to natural or artificial factors. For example, the unmodified microorganism may be a strain into which the geranylgeranyl pyrophosphate synthase variant of the present disclosure is not introduced or has not yet been introduced. The term "unmodified microorganism" may be used interchangeably with "strain before being modified", "strain before being modification", "microorganism before being modified", "microorganism before being modification", "unvaried strain", "unmodified strain", "unvaried microorganism", or "reference microorganism".

In one embodiment, the microorganism of the present disclosure may be a microorganism belong to the *Yarrowia* sp., and specifically, *Yarrowia lipolytica,* but is not limited thereto.

As used herein, the term "enhancement" of polypeptide activity means that the activity of a polypeptide is increased, as compared to the endogenous activity. The enhancement may be used interchangeably with terms such as activation, up-regulation, overexpression, and increase, *etc.* Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of the polypeptide is improved as compared to the activity and/or concentration (expression level) of a specific polypeptide originally possessed by a parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the corresponding polypeptide or in the amount of the product produced from the corresponding polypeptide.

For the enhancement of the activity of the polypeptide, various methods well known in the art may be applied, and the method is not limited as long as the activity of the interest polypeptide may be enhanced as compared to that of the microorganism before being modified. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.)*.*

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a nucleotide sequence encoding an initiation codon or 5'-UTR region of a gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically:
1) The increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing a vector capable of inserting the polynucleotide into a chromosome of a host cell into the host cell, but is not limited thereto. The vector is as described above.
2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may include, but is not particularly limited to, a promoter, an operator sequence, a sequence encoding a ribosome binding site, a sequence controlling the termination of transcription and translation, *etc.* For example, the replacement may be to replace the original promoter with a strong promoter, but is not limited thereto.
   Examples of known strong promoters include cj1 to cj7 promoters (US Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. US 10584338 B2), O2 promoter (US Patent No. US 10273491 B2), tkt promoter, yccA promoter, *etc.,* but is not limited thereto.
3) The modification of a nucleotide sequence encoding an initiation codon or 5'-UTR region of a gene transcript encoding the polypeptide may be, for example, substitution with a nucleotide sequence encoding another start codon having a higher polypeptide expression rate as compared to an endogenous start codon, but is not limited thereto.
4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further include a selection marker for the confirmation of chromosome insertion. The selection marker is as described above.
6) The introduction of a foreign polynucleotide exhibiting the activity of the polypeptide may be introduction of a foreign polynucleotide encoding a polypeptide exhibiting activity identical or similar to that of the polypeptide into a host cell. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in a host cell, the polypeptide may be produced, and the activity thereof may be increased.
7) The codon optimization of the polynucleotide encoding the polypeptide may be codon optimization of an endogenous polynucleotide so as to increase transcription or translation in a host cell, or codon optimization of a foreign polynucleotide so as to perform optimized transcription and translation in a host cell.
8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type or a microbial strain before being modified, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may include a method using a DNA recombination technology. For example, by introducing a nucleotide sequence or vector including a nucleotide sequence homologous to the gene of interest into the microorganism to cause homologous recombination, a part or the entirety of the gene may be deleted. The nucleotide sequence or vector to be introduced may include a dominant selection marker, but is not limited thereto.

Still another aspect of the present disclosure provides a method of producing tetraterpene, a precursor of tetraterpene, and a material having tetraterpene as a precursor, the method including culturing, in a medium, the microorganism including any one or more of the variant of the present disclosure; the polynucleotide encoding the variant; and the vector including the polynucleotide. The method of producing the tetraterpene, the precursor of tetraterpene, and the material having tetraterpene as a precursor may be a method of reducing squalene production, but is not limited thereto.

As used herein the term "culture" refers to growing the microorganism of the present disclosure in appropriately adjusted environment conditions. The culture procedure of the present disclosure may be performed according to appropriate media or culture conditions known in the art. Such culture may be easily adjusted according to the selected microorganism by a person skilled in the art. Specifically, the culture may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials including water essential for survival and growth, growth factors, *etc.* Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, *etc.*

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* and amino acids, such as glutamic acid, methionine, lysine, *etc.* In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, *etc.* may be used. These nitrogen sources may be used alone or in a combination of two or more thereof, but are not limited thereto.

The phosphate sources may include potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, *etc.* may be used, and in addition, amino acids, vitamins, and/or suitable precursors may be included. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be added to suppress foam formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, 20°C to 35°C, or 25 to 35°C, and the culture may be performed for about 10 hours to 160 hours, but is not limited thereto.

The tetraterpene, the precursor of tetraterpene, or the material having tetraterpene as a precursor which is produced by the culture of the present disclosure may be released into the medium or may remain in cells.

The method of producing the tetraterpene, the precursor of tetraterpene, or the material having tetraterpene as a precursor of the present disclosure may further include preparing the microorganism of the present disclosure, preparing a medium for culturing the microorganism, or a combination thereof regardless of the order, in any order), for example, before or after the culturing.

The method of producing the tetraterpene, the precursor of tetraterpene, or the material having tetraterpene as a precursor of the present disclosure may further include recovering the tetraterpene, the precursor of tetraterpene, or the material having tetraterpene as a precursor from a medium resulting from the culture (a medium in which culture has been performed) or from the microorganism of the present disclosure. The recovering may be further included after the culturing.

The recovering may be collecting the desired tetraterpene, precursor of tetraterpene, or material having tetraterpene as a precursor by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, cell disruption, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and the desired tetraterpene, precursor of tetraterpene, or material having tetraterpene as a precursor may be recovered from the medium or microorganism by using an appropriate method known in the art.

In addition, the method of producing the tetraterpene, the precursor of tetraterpene, or the material having tetraterpene as a precursor of the present disclosure may further include purification. The purification may be performed by using an appropriate method known in the art. In an exemplary embodiment, when the method of producing the tetraterpene, the precursor of tetraterpene, or the material having tetraterpene as a precursor of the present disclosure includes both the recovering and the purification, the recovering and the purification may be performed discontinuously (or continuously) regardless of the order, or may be performed simultaneously or integrated into one step, but is not limited thereto.

The method of producing the material having tetraterpene as a precursor of the present disclosure may further include the converting tetraterpene into the material having tetraterpene as a precursor. In the method of producing the material having tetraterpene as a precursor of the present disclosure, the converting may be further included after the culturing or the recovering. The converting may be performed using an appropriate method known in the art. For example, the converting may be performed using beta-carotene 15,15'-oxygenase or retinol dehydrogenase, but is not limited thereto.

The variant, polynucleotide, vector, microorganism, tetraterpene, precursor of tetraterpene, and material having tetraterpene as a precursor are as described above.

Still another aspect of the present disclosure provides a composition for producing the tetraterpene, the precursor thereof, and the material having tetraterpene as a precursor, the composition including the variant of the present disclosure; the vector of the present disclosure; the microorganism including the variant of the present disclosure, the polynucleotide encoding the variant, the vector including the polynucleotide, or the polynucleotide of the present disclosure; the culture of the microorganism; or a combination of two or more thereof.

The composition of the present disclosure may further include any appropriate excipient that is usually used in the composition for producing the tetraterpene, the precursor thereof, and the material having tetraterpene as a precursor, and examples of the excipient may include a preserving agent, a wetting agent, a dispersing agent, a suspending agent, a buffer, a stabilizing agent, an isotonic agent, *etc.,* but are not limited thereto.

The variant, polynucleotide, vector, microorganism, medium, tetraterpene, precursor of tetraterpene, and material having tetraterpene as a precursor are as described above.

Still another aspect of the present disclosure provides use of any one or more of the variant, polynucleotide, vector, and microorganism of the present disclosure, for producing the tetraterpene, the precursor of tetraterpene, and the material having tetraterpene as a precursor.

The variant, polynucleotide, vector, microorganism, tetraterpene, precursor of tetraterpene, and material having tetraterpene as a precursor are as described above.

### [Mode for Carrying Out the Invention]

Hereinafter, the present disclosure will be described in more detail with reference to Examples. However, these Examples and Experimental Examples are only for illustrating the present disclosure, and the scope of the present disclosure is not intended to be limited by these Examples and Experimental Examples.

### Example 1. Construction of GGS1 variant vector libraries

To amplify GGS1 gene encoding geranylgeranyl pyrophosphate synthase on the *Yarrowia lipolytica* chromosome, an amino acid sequence of SEQ ID NO: 1 and a polynucleotide sequence of SEQ ID NO: 2 of GGS1 (YALI0D17050) were obtained based on a nucleotide sequence registered in Kyoto Encyclopedia of Genes and Genomes (KEGG). A diversify PCR random mutagenesis kit (Takara) was used to induce random mutations through error-prone PCR (ep-PCR). PCR conditions and methods were optimized according to buffer condition 1 and buffer condition 2 in the user manual, respectively. Specific error-prone PCR conditions are shown in Table 1 below.

**[Table 1]**

| | Buffer condition 1 | Buffer condition 2 |
|---|---|---|
| PCR grade water | 40 µl | 39 µl |
| 10X Titanium Taq buffer | 5 µl | 5 µl |
| MnSO4 | 0 µl | 1 µl |
| dGTP | 1 µl | 1 µl |
| 50X Diversify dNTP Mix | 1 µl | 1 µl |
| Primer mix | 1 µl | 1 µl |
| Template DNA | 1 µl | 1 µl |
| Titanium Taq polymerase | 1 µl | 1 µl |

PCR was performed using *Yarrowia lipolytica* PO1f genomic DNA as a template and primers of SEQ ID NO: 19 and SEQ ID NO: 20. PCR conditions were as follows: 32 cycles consisting of denaturation at 95°C for 1 minute; annealing at 55°C for 1 minute; and polymerization at 68°C for 1 minute. A GGS1 variant-expressing vector library based on a pIMR53-EXP1p-CYC1t vector was created by mixing GGS1 PCR products of two conditions which were amplified by PCR.

The pIMR53-EXP1p-CYC1t vector was constructed as follows. To amplify EXP1p, PCR was performed using *Yarrowia lipolytica* PO1f genomic DNA as a template and primers of SEQ ID NO: 21 and SEQ ID NO: 22, and to amplify a CYC1t terminator, PCR was performed using *Saccharomyce cerevisiae* genomic DNA as a template and primers of SEQ ID NO: 23 and SEQ ID NO: 24. PCR conditions were as follows: 32 cycles consisting of denaturation at 95°C for 1 minute; annealing at 55°C for 1 minute; and polymerization at 68°C for 1 minute. Additionally, PCR was performed using a pIMR53 vector as a template and primers of SEQ ID NO: 25 and SEQ ID NO: 26. PCR conditions were as follows: 32 cycles consisting of denaturation at 95°C for 1 minute; annealing at 55°C for 1 minute; and polymerization at 68°C for 5 minutes.

As a result, 6,774 bp of linearized pIMR53, 1,050 bp of EXP1p, and 289 bp of CYC1t were obtained. The EXP1p and CYC1t DNA fragments amplified by PCR were conjugated to the pIMR53 vector using an Infusion cloning kit (Invitrogen), transformed into E. *coli* DH5α, and plated on LB solid medium containing 30 mg/L ampicillin. After selecting colonies transformed with the plasmid into which the target EXP1p promoter and CYC1t terminator were inserted through PCR, a plasmid was obtained using a commonly known plasmid extraction method, and this plasmid was named pIMR53-EXP1p-CYC1t.

### Example 2. Preparation of Yarrowia lipolytica platform strain for beta-carotene production

### Example 2-1. Preparation of X. dendrorhous-derived crtYB-crtl inserted strain

To prepare a platform strain for beta-carotene production, lycopene cyclase/phytoene synthase (crtYB) and phytoene desaturase(crtl) genes derived from *Xanthophyllomyces dendrorhous* were inserted into the genome of a high-fat yeast *Yarrowia lipolytica* CC08-0125 (Accession No. KCCM12972P) strain. With regard to crtYB, a polynucleotide of SEQ ID NO: 27 was obtained, based on a nucleotide sequence (GenBank: AY177204.1) registered in National Center for Biotechnology Information Search database (NCBI), and with regard to crtl, a polynucleotide of SEQ ID NO: 28 was obtained, based on a nucleotide sequence (GenBank: AY177424.1) registered in NCBI. The polynucleotide sequences of crtYB and crtl were synthesized by Macrogen Inc. in the form of TEFINtp-crtYB-CYC1t (SEQ ID NO: 29) and TEFINtp-crtl-CYC1t (SEQ ID NO: 30), respectively, and a cassette inserted into the site of MHY1 (YALI0B21582g) gene was designed using a URA3 gene (SEQ ID NO: 31) of *Y. lipolytica* as a selection marker. PCR was performed using the synthesized crtYB, crtl gene and KCCM12972P genomic DNA as templates and primers of SEQ ID NO: 32 and SEQ ID NO: 33, SEQ ID NO: 34 and SEQ ID NO: 35, SEQ ID NO: 36 and SEQ ID NO: 37, SEQ ID NO: 38 and SEQ ID NO: 39, SEQ ID NO: 40 and SEQ ID NO: 41, SEQ ID NO: 42 and SEQ ID NO: 43, respectively. PCR conditions were as follows: 35 cycles consisting of denaturation at 95°C for 1 minute; annealing at 55°C for 1 minute; and polymerization at 72°C for 3 minutes and 30 seconds. The resulting five DNA fragments were prepared as a single cassette through overlap extension PCR.

The cassette thus prepared was introduced into strain KCCM12972P by a heat shock method (D.-C. Chen et al., Appl Microbiol Biotechnol, 1997), and then colonies formed on a solid medium (YLMM1) without uracil were obtained. Colonies, in which insertion of the cassette into the genome was confirmed using primers of SEQ ID NO: 44 and SEQ ID NO: 45, were spotted on a 5-FOA solid medium and cultured at 30°C for 3 days. The URA3 marker was recovered by obtaining the colonies grown on the 5-FOA solid medium.

### Example 2-2. Preparation of HMGR-enhanced strain

A cassette was designed to replace, with a TEFINt promoter, a native promoter (SEQ ID NO: 46) region of 3-hydroxy-3-methylglutaryl-CoA reductase (HMGR) gene of the strain prepared in Example 2-1, into which the crtYB and crtl genes were inserted. PCR was performed using KCCM12972P genomic DNA as a template, and primers of SEQ ID NO: 47 and SEQ ID NO: 48, SEQ ID NO: 49 and SEQ ID NO: 50, SEQ ID NO: 51 and SEQ ID NO: 52, SEQ ID NO: 53 and SEQ ID NO: 54, SEQ ID NO: 55 and SEQ ID NO: 56, respectively. PCR conditions were as follows: 35 cycles consisting of denaturation at 95°C for 1 minute; annealing at 55°C for 1 minute; and polymerization at 72°C for 1 minute and 30 seconds. The resulting five DNA fragments were prepared as a single cassette through overlap extension PCR.

The cassette thus prepared was introduced into the strain prepared in Example 2-1 by a heat shock method, and then colonies formed on a solid medium (YLMM1) without uracil were obtained. Colonies, in which insertion of the cassette was confirmed using primers of SEQ ID NO: 57 and SEQ ID NO: 58, were spotted on a 5-FOA solid medium and cultured at 30°C for 3 days. The URA3 marker was recovered by obtaining the colonies grown on the 5-FOA solid medium. The beta-carotene-producing platform strain finally prepared was named CC08-1023.

### <Yarrowia lipolytica Minimal Media 1 (YLMM1)>

20 g/L of glucose, 6.7 g/L of yeast nitrogen base without amino acids, 2 g/L of yeast synthetic drop-out medium supplements without uracil, and 15 g/L of agar

### <5-Fluoroorotic Acid (5-FOA)>

20 g/L of glucose, 6.7 g/L of yeast nitrogen base without amino acids, 2 g/L of yeast synthetic drop-out medium supplements without uracil, 50 µg/mL of uracil, 1 g/L of 5-Fluoroorotic acid (5-FOA), and 15 g/L of agar

### Example 3. Primary selection of GGS1 activity-enhanced strain

For primary selection of the GGS1 activity-enhanced strains, color-based primary selection was performed.

First, pIMR53 (negative control; NC), pIMR53-EXP1p-GGS1 (wild-type, SEQ ID NO: 2)-CYC1t vector (positive control; PC), and the GGS1 random variant-expressing vector library constructed in Example 1 were transformed into *Yarrowia lipolytica* CC08-1023 strain producing a small amount of beta-carotene by heat-shock, respectively. Thereafter, colonies formed on a solid medium without uracil were obtained.

350 µl of a liquid medium of *Yarrowia lipolytica* minimal media 2 (YLMM2) without uracil was dispensed into each well of 96 deep-well plates (25 plates), and the obtained colonies were inoculated and cultured at 30°C for 48 hours. Thereafter, they were inoculated on a YLMM1 solid medium without uracil, and cultured at 30°C for 3 days. After the end of the culture, 36 kinds of orange-colored strains, which were darker in color as compared to the strain transformed with the positive control (CC08-1023/pIMR53-EXP1p-GGS1-CYC1t) vector, were first selected from a total of 2,400 kinds of strains. The method of preparing the above described YLMM2 and the composition thereof are as follows.

### <YLMM2 (pH 7.2)>

40 g/L of glucose, 1.7 g/L of yeast nitrogen base without amino acids and ammonium sulfate, 0.5 g/L of uracil, and 2.5 g/L of ammonium sulfate dissolved in 0.1 M sodium phosphate buffer (pH 7.2)

### Example 4. Secondary selection of GGS1 activity-enhanced strain

For secondary selection of the GGS1 activity-enhanced strains, a flask test was conducted on 36 kinds of strains first selected in Example 3.

In a 250 ml corner-baffled flask containing 50 ml of *Yarrowia lipolytica* minimal media 2 (YLMM2) without uracil, 36 kinds of strains and the strain transformed with the positive control (PC) vector were inoculated at an initial OD of 1, respectively and cultured at 30°C for 48 hours with shaking at 250 rpm.

After completion of the culture, 1 ml of the culture medium was centrifuged and the supernatant was discarded. Next, 0.5 ml of dimethyl sulfoxide (DMSO, Sigma) was added, and the cells were disrupted by shaking at 2,000 rpm for 10 minutes at 55°C. Additionally, 0.5 ml of acetone (Sigma) was added and shaken at 2,000 rpm for 15 minutes at 45°C to extract beta-carotene and squalene. The extracted materials were analyzed for concentration using HPLC equipment. The results of the flask test on the selected strains, and the results of measuring OD, concentrations and contents of beta-carotene and squalene are shown in Table 2 below.

**[Table 2]**

| Numb. | OD | β-carotene(mg/L) | Squalene(mg/L) | β-carotene(%) | Squalene(%) |
|---|---|---|---|---|---|
| PC | 60 | 38 | 248 | 0.25 | 1.66 |
| 1 | 59 | 54 | 154 | 0.36 | 1.04 |
| 2 | 61 | 35 | 214 | 0.23 | 1.41 |
| 3 | 65 | 62 | 212 | 0.38 | 1.32 |
| 4 | 50 | 30 | 102 | 0.24 | 0.82 |
| 5 | 62 | 61 | 194 | 0.39 | 1.24 |
| 6 | 62 | 55 | 282 | 0.36 | 1.83 |
| 7 | 55 | 46 | 173 | 0.33 | 1.27 |
| 8 | 48 | 40 | 180 | 0.34 | 1.52 |
| 9 | 56 | 34 | 132 | 0.25 | 0.95 |
| 10 | 50 | 46 | 185 | 0.36 | 1.48 |
| 11 | 51 | 41 | 120 | 0.33 | 0.94 |
| 12 | 62 | 52 | 218 | 0.34 | 1.42 |
| 13 | 63 | 57 | 210 | 0.37 | 1.34 |
| 14 | 59 | 53 | 230 | 0.36 | 1.57 |
| 15 | 58 | 57 | 185 | 0.39 | 1.29 |
| 16 | 53 | 40 | 219 | 0.31 | 1.66 |
| 17 | 60 | 60 | 121 | 0.40 | 0.81 |
| 18 | 59 | 53 | 206 | 0.36 | 1.40 |
| 19 | 62 | 50 | 207 | 0.32 | 1.33 |
| 20 | 62 | 59 | 189 | 0.38 | 1.22 |
| 21 | 60 | 69 | 108 | 0.46 | 0.72 |
| 22 | 59 | 60 | 148 | 0.41 | 1.00 |
| 23 | 62 | 58 | 134 | 0.37 | 0.86 |
| 24 | 60 | 57 | 198 | 0.38 | 1.33 |
| 25 | 64 | 48 | 214 | 0.30 | 1.33 |
| 26 | 67 | 56 | 228 | 0.34 | 1.37 |
| 27 | 59 | 47 | 214 | 0.32 | 1.45 |
| 28 | 61 | 35 | 183 | 0.23 | 1.20 |
| 29 | 60 | 51 | 219 | 0.34 | 1.45 |
| 30 | 59 | 48 | 201 | 0.33 | 1.38 |
| 31 | 64 | 61 | 198 | 0.38 | 1.23 |
| 32 | 65 | 66 | 169 | 0.40 | 1.04 |
| 33 | 59 | 47 | 206 | 0.32 | 1.40 |
| 34 | 62 | 51 | 238 | 0.32 | 1.53 |
| 35 | 53 | 37 | 189 | 0.28 | 1.42 |
| 36 | 61 | 53 | 225 | 0.35 | 1.47 |

Thereafter, the concentrations of beta-carotene and squalene extracted from the test samples were divided by DCW (g/L), which were used as a standard for secondary selection. It was confirmed that the value (β-carotene (%)) of the beta-carotene concentration divided by DCW (g/L) was increased in 33 kinds of test strains, excluding Strain No. 2, No. 4, No. 9, and No. 28, as compared to the positive control (PC). It was confirmed that the value of the squalene concentration divided by DCW (g/L) was reduced in all test strains, as compared to the positive control (PC).

Therefore, reference values were determined, based on the values calculated from the positive control (PC), and strains corresponding to both conditions were selected, and a total of four candidate groups corresponding to both condition 1 and condition 2 were selected. The reference values for secondary selection and four candidates secondarily selected are shown in Table 3 below.

**[Table 3]**

| Condition | Reference | Selection results (flask no.) | Note |
|---|---|---|---|
| #1 | β-carotene content (%) ≥ 0.38 | 3, 5, 15, 17, 20, 21, 22, 24, 31, 32 | GGS1(PC)=0.2 5 |
| #2 | Squalene content (%) < 1.05 | 2, 4, 9, 11, 17, 21, 22, 23, 32 | GGS1(PC)=1.6 6 |
| Condition 1 + Condition 2 | | 17, 21, 22, 32 | |

The transformed plasmids were extracted from the four kinds of selected strains, and sequence analysis was performed to confirm random mutations located on the GGS1 ORF. The transformed GGS1 plasmid was extracted from the selected strain using a Zymoprep yeast plasmid miniprep kit 2 (Zymo research), and in order to increase reliability of the sequence analysis, bidirectional sequencing was performed using primers of SEQ ID NO: 59 and SEQ ID NO: 60. The amino acid mutations which were identified as a result of the sequence analysis of GGS1 ORF are shown in Table 4 below, and the four kinds of candidate groups were confirmed to have one mutation or two mutations among those in Table 4 below.

**[Table 4]**

| Variant flask no. | Genotype |
|---|---|
| 17 | GGS1(I122V, R141K) |
| 21 | GGS1(N29T, L90R) |
| 22 | GGS1(M103I) |
| 32 | GGS1(F43I) |

### Example 5. Verification of activity of selected GGS1 variant

An experiment was performed to verify the activities of the GGS1 variants selected in Example 3 and Example 4.

To this end, a flask test was performed on a total of 10 kinds of strains which were prepared by transforming each expressing negative control (NC) and positive control (PC) vectors and expression vectors for four kinds of variants (M103I variant, F43I variant, N29T and L90R variant, and I122V and R141K variant) selected in Example 4, and four kinds of related single variants (N29T variant, L90R variant, 1122V variant, and R141K variant).

The method of preparing expression vectors for each of the four kinds of single variants (N29T variant, L90R variant, 1122V variant, and R141K variant) is as follows. To obtain DNA fragments of the four kinds of single variants (N29T variant, L90R variant, 1122V variant, and R141K variant), PCR was performed using the pIMR53-EXP1p-GGS1-CYC1t vector containing the wild-type GGS1 sequence as a template, and primers of SEQ ID NO: 61 and SEQ ID NO: 62, SEQ ID NO: 63 and SEQ ID NO: 64, SEQ ID NO: 65 and SEQ ID NO: 66, SEQ ID NO: 67 and SEQ ID NO: 68, respectively. PCR conditions were as follows: 32 cycles consisting of denaturation at 95°C for 1 minute; annealing at 55°C for 1 minute; and polymerization at 68°C for 5 minutes. The expression vectors for the N29T and L90R variant or the 1122V and R141K variant which are the double variant were prepared by performing PCR using the N29T expression vector or I122V expression vector prepared above as a template and the primer of SEQ ID NO: 63 and SEQ ID NO: 64 and the primer of SEQ ID NO: 67 and SEQ ID NO: 68 in the same manner.

As a result, the variant-introduced DNA amplified by PCR was transformed into E. *coli* DH5α using an Infusion cloning kit (Invitrogen), and plated on LB solid medium containing 30 mg/L of ampicillin. After selecting colonies which were transformed with plasmids into which four kinds of transformed GGS1 variant vectors were inserted, plasmids were obtained using a commonly known plasmid extraction method, and sequences of the variants were confirmed using SEQ ID NO: 59 and SEQ ID NO: 60.

In detail, in a 250 ml corner-baffled flask containing 50 ml of *Yarrowia lipolytica* minimal media 2 (YLMM2) without uracil, 10 kinds of strains were inoculated at an initial OD of 1, respectively and cultured at 30°C for 48 hours with shaking at 250 rpm.

After completion of the culture, 1 ml of the culture medium was centrifuged and the supernatant was discarded. Next, 0.5 ml of dimethyl sulfoxide (DMSO, Sigma) was added, and the cells were disrupted by shaking at 2,000 rpm for 10 minutes at 55°C. Additionally, 0.5 ml of acetone (Sigma) was added and shaken at 2,000 rpm for 15 minutes at 45°C to extract beta-carotene and squalene. Their concentrations were analyzed using HPLC equipment. The results of measuring OD, concentrations and contents of beta-carotene and squalene are shown in Table 5 below.

**[Table 5]**

| Genotype | OD | β-carotene (mg/L) | Squalene (mg/L) | β-carotene (%) | Squalene (%) |
|---|---|---|---|---|---|
| NC | 62 | 5 | 208 | 0.03 | 1.34 |
| PC | 56 | 30 | 133 | 0.21 | 0.95 |
| M103I | 62 | 59 | 91 | 0.38 | 0.59 |
| F43I | 63 | 69 | 71 | 0.44 | 0.45 |
| N29T/L90R | 64 | 67 | 89 | 0.42 | 0.56 |
| N29T | 61 | 55 | 88 | 0.36 | 0.58 |
| L90R | 64 | 58 | 90 | 0.36 | 0.56 |
| I122V/R141K | 62 | 62 | 65 | 0.40 | 0.42 |
| 1122V | 57 | 43 | 118 | 0.30 | 0.83 |
| R141K | 59 | 44 | 111 | 0.30 | 0.75 |

As shown in Table 5, it was confirmed that the beta-carotene concentration increased by 143% to 230% and the squalene concentration decreased by 11% to 51% in all the selected M103I, F43I, N29T, L90R, I122V, and/or R141K mutation-applied GGS1 variants, as compared to the positive control (PC). Consequently, among 6 kinds of strains into which the single variant was introduced, the strain into which the F43I variant was introduced showed the best effect. In the case of the I122V and R141K variants, the effects of increasing the beta-carotene concentration and decreasing the squalene production were further enhanced when applying both the variants simultaneously, as compared to applying the variants alone. In the case of the N29T and L90R variants, the effect of decreasing the squalene production was further enhanced when applying both the variants simultaneously, as compared to applying the variants alone.

### Example 6. Preparation and Evaluation of GGS1 variant-introduced strain

It was intended to verify the effect when the GGS1 F43I variant, which was determined to be the most excellent in the previous Example, was actually introduced into a strain.

To this end, based on CC08-1023, a strain (CC08-1214) in which the promoter of the GGS1 gene was replaced and a strain (CC08-1215) expressing a variant in which phenylalanine at position 43 of GGS1 was replaced with isoleucine were sequentially prepared.

### Example 6-1. Preparation of GGS1 promoter-replaced strain ("CC08-1214")

To construct a cassette that is able to replace the GGS1 promoter with the TEFINt promoter on the *Yarrowia lipolytica* chromosome, a polynucleotide sequence of SEQ ID NO: 69 containing the promoter of TEF1 (YALI0C09141p) and splicing points was obtained, based on the nucleotide sequence registered in Kyoto Encyclopedia of Genes and Genomes (KEGG). In addition, for replacement of the promoter of GGS1, a polynucleotide sequence of SEQ ID NO: 2 of GGS1 (YALI0D17050g) was obtained. PCR was performed using *Yarrowia lipolytica* PO1f genomic DNA as a template, and primers of SEQ ID NO: 70 and SEQ ID NO: 71, SEQ ID NO: 72 and SEQ ID NO: 73, SEQ ID NO: 74 and SEQ ID NO: 75, and SEQ ID NO: 76 and SEQ ID NO: 77, respectively. Then, PCR was performed using a URA3 auxotrophic marker as a template and primers of SEQ ID NO: 78 and SEQ ID NO: 79. PCR conditions were as follows: 35 cycles consisting of denaturation at 95°C for 1 minute; annealing at 55°C for 1 minute; and polymerization at 72°C for 1 minute and 30 minutes.

As a result, 1,500 bp of a 5'UTR arm, 531 bp of 5'UTR_RP, 1,500 bp of GGS1 arm, and 1,533 bp of URA3 were obtained. The DNA fragments amplified by PCR were prepared into a single GGS1 promoter replacement cassette through overlap extension PCR, and the design of cassette was made in the following order of 5'UTR arm-TEFINt-URA3-TEFINt-GGS1 arm.

The GGS1 promoter replacement cassette was transformed into CC08-1023 by heat-shock and selected on YLMM1 solid medium without uracil. The selected primary strains were subjected to a secondary crossover to finally prepare a strain in which the promoter of the GGS1 gene was replaced with the TEFINt promoter. The strain thus prepared was named "CC08-1214".

### Example 6-2. Preparation of GGS1(F43I) variant strain ("CC08-1215")

To introduce the GGS1 protein F43I variant-expressing sequence into the *Yarrowia lipolytica* chromosome, a cassette was constructed in which thymine at position 127 of the polynucleotide sequence of SEQ ID NO: 2 was replaced with adenine.

In detail, PCR was performed using the *Yarrowia lipolytica* PO1f genomic DNA as a template and primers of SEQ ID NO: 80 and SEQ ID NO: 81, SEQ ID NO: 82 and SEQ ID NO: 83, SEQ ID NO: 84 and SEQ ID NO: 85, SEQ ID NO: 86 and SEQ ID NO: 83, SEQ ID NO: 84 and SEQ ID NO: 87, and SEQ ID NO: 90 and SEQ ID NO: 91, respectively. Further, PCR was performed using the URA3 auxotrophic marker as a template and primers of SEQ ID NO: 88 and SEQ ID NO: 89. PCR conditions were as follows: 35 cycles consisting of denaturation at 95°C for 1 minute; annealing at 55°C for 1 minute; and polymerization at 72°C for 1 minute and 30 minutes.

As a result, 1,500 bp of a 5'UTR arm, 661 bp of TEFINt-GGS1_1, 1,390 bp of GGS1_2, 661 bp of RP_1, 1,390 bp of RP_2, 1,500 bp of 3'UTR arm, and 1,533 bp of URA3 were obtained. The DNA fragments amplified by PCR were prepared into a single GGS1 promoter replacement cassette through overlap extension PCR, and the design of cassette was made in the order of 5'UTR arm- TEFINt-GGS1_1-GGS1_2- RP_1- RP_2-3'UTR arm.

The GGS1 (F43I) variant replacement cassette was transformed into CC08-1214 by heat-shock and selected on YLMM1 solid medium without uracil. The selected primary strains were subjected to a secondary crossover to finally prepare a F43I variant-expressing strain in which the amino acid residue at position 43 of GGS1 was replaced with isoleucine. The strain thus prepared was named "CC08-1215".

### Example 6-3. Evaluation of GGS1 variant-introduced strain

A flask test was performed on CC08-1215 together with CC08-1023 as a negative control and CC08-1214 as a positive control.

In detail, CC08-1215, CC08-1023 (negative control), and CC08-1214 (positive control) were inoculated to a 250 ml corner-baffled flask containing 50 ml of *Yarrowia lipolytica* minimal media 2 (YLMM2) with uracil at an initial OD of 1, and cultured at 30°C for 48 hours with shaking at 250 rpm, respectively.

After completion of the culture, 1 ml of the culture medium was centrifuged and the supernatant was discarded. Thereafter, 0.5 ml of dimethyl sulfoxide (DMSO, Sigma) was added, and the cells were disrupted by shaking at 2,000 rpm for 10 minutes at 55°C. Additionally, 0.5 ml of acetone (Sigma) was added and shaken at 2,000 rpm for 15 minutes at 45°C to extract beta-carotene and squalene. Their concentrations were analyzed using HPLC equipment. The analyzed OD, and the results of measuring concentrations and contents of beta-carotene and squalene are shown in Table 6 below.

**[Table 6]**

| Genotype | OD | β-carotene (mg/L) | Squalene (mg/L) | β-carotene (%) | Squalene (%) |
|---|---|---|---|---|---|
| CC08-1023(NC) | 46 | 5 | 41 | 0.04 | 0.36 |
| CC08-1214(PC) | 50 | 32 | 17 | 0.26 | 0.14 |
| CC08-1215 | 48 | 49 | 2 | 0.41 | 0.02 |

As shown in Table 6, it was confirmed that the beta-carotene concentration increased by 51.3% and the squalene concentration decreased by 88.5% in the selected F43I variant-applied CC08-1215 strain, as compared to CC08-1214(PC). Consequently, it was confirmed that the effect of introducing the F43I single variant had the effects of increasing the beta-carotene concentration and decreasing the squalene production even when actually applied to the strain.

These results confirmed that the beta-carotene production may be increased by introducing the GGS1 variant of the present disclosure. It was also confirmed that the production efficiency of beta-carotene may be further enhanced by reducing the squalene synthesis, which is a competing pathway for beta-carotene production, due to introduction of the GGS1 variant.

The above results also confirmed that, like the beta-carotene production, the production efficiency of tetraterpenes involving GGS1 may be further enhanced.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0125 | Accession number given by the
INTERNATIONAL DEPOSITARY AUTHORITY:
KCCM12972P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 09. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 09. 2021.
한국미생
물보존센
터장의인

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩 Tel: 02-391-0950, 396-0950 Fax: 02-392-2859
KOREAN CULTURE CENTER OF MICROORGANISMS [?]
Yurim Bldg., 45, Hongjenae-2ga-gil, Seodaemun-gu, Seoul, 03641, Korea. Tel: 82-2-391-0950, 396-0950 Fax: 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR:
Yarrowia lipolytica CC08-0125 | Accession number given by the
INTERNATIONAL DEPOSITARY AUTHORITY:
KCCM12972P

II. SCIENTIFIC DESCRIPTION AND/OR PROPOSED TAXONOMIC DESIGNATION

The microorganism identified under I above was accompanied by:
☐ a scientific description
☐ a proposed taxonomic designation
(Mark with a cross where applicable)

III. RECEIPT AND ACCEPTANCE

This International Depositary Authority accepts the microorganism identified under I above,
which was received by it on April. 09. 2021 (date of the original deposit).¹

IV. RECEIPT OF REQUEST FOR CONVERSION

The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY

Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 09. 2021.

¹ Where Rule 6.4(d) applies, such date is the date on which the status of international depositary authority was
acquired.

Form BP/4 (sole page)
한국미생물보존센터 [?]
03641 서울특별시 서대문구 홍제내2가길 45 유림빌딩 Tel: 02-391-0950 396-0950 Fax: 02-392-2859
KOREAN CULTURE CENTER OF MICROORGANISMS [?]
Yurim Bldg., 45, Hongjenae 2ga-gil, Seodaemun-gu, Seoul 03641, Korea. Tel: 82-2-391-0950 396-0950 Fax: 82-2-392-2859

## Claims

1. A geranylgeranyl pyrophosphate synthase variant, wherein any one or more of amino acids corresponding to positions 29, 43, 90, 103, 122, and 141 from the N-terminus of an amino acid sequence of SEQ ID NO: 1 are substituted with another amino acid.

2. The geranylgeranyl pyrophosphate synthase variant of claim 1, wherein the variant includes one or more substitutions of a substitution of the amino acid corresponding to position 29 from the N-terminus of the amino acid sequence of SEQ ID NO: 1 with an amino acid other than asparagine; a substitution of the amino acid corresponding to position 43 with an amino acid other than phenylalanine; a substitution of the amino acid corresponding to position 90 with an amino acid other than leucine; a substitution of the amino acid corresponding to position 103 with an amino acid other than methionine; a substitution of the amino acid corresponding to position 122 with an amino acid other than isoleucine; and a substitution of the amino acid corresponding to position 141 with an amino acid other than arginine.

3. The geranylgeranyl pyrophosphate synthase variant of claim 1, wherein the substitution with another amino acid is substitution with a nonpolar amino acid, a polar amino acid, or a positively charged (basic) amino acid.

4. The geranylgeranyl pyrophosphate synthase variant of claim 1, wherein another amino acid is any one selected from the group consisting of threonine (Thr), isoleucine (Ile), arginine (Arg), valine (Val), and lysine (Lys).

5. A polynucleotide encoding the geranylgeranyl pyrophosphate synthase variant of any one of claims 1 to 4.

6. A microorganism comprising any one or more of the geranylgeranyl pyrophosphate synthase variant of any one of claims 1 to 4 and a polynucleotide encoding the variant.

7. The microorganism of claim 6, wherein the microorganism produces tetraterpene, a precursor of tetraterpene, or a material having tetraterpene as a precursor.

8. The microorganism of claim 6, wherein the microorganism belongs to the *Yarrowia* sp.

9. The microorganism of claim 6, wherein the microorganism is *Yarrowia lipolytica.*

10. The microorganism of claim 6, wherein the microorganism produces beta-carotene, a precursor of beta-carotene, or a material having beta-carotene as a precursor.

11. A method of producing tetraterpene, a precursor of tetraterpene, or a material having tetraterpene as a precursor, the method comprising culturing the microorganism of claim 6 in a medium.

12. The method of claim 11, further comprising recovering the tetraterpene, the precursor of tetraterpene, or the material having tetraterpene as a precursor from the medium or from the microorganism.

13. The method of claim 11, wherein the microorganism belongs to the *Yarrowia* sp.

14. The method of claim 11, wherein the method reduces squalene production.

15. A composition for producing tetraterpene, a precursor thereof, or a material having tetraterpene as a precursor, the composition comprising the variant of claim 1, the microorganism of claim 6, or a culture of the microorganism.

16. Use of a microorganism comprising any one or more of a geranylgeranyl pyrophosphate synthase variant, wherein any one or more of amino acids corresponding to positions 29, 43, 90, 103, 122, and 141 from the N-terminus of an amino acid sequence of SEQ ID NO: 1 are substituted with another amino acid; and a polynucleotide encoding the variant, for producing tetraterpene, a precursor thereof, or a material having tetraterpene as a precursor.
